# EUROPEAN PATENT APPLICATION

(11) **EP 1 684 172 A1**
(43) Date of publication of application: **26.07.2006**
(21) Application number: 05075179.1
(22) Date of filing: 24.01.2005
(51) Int. Cl.: G06F 9/445

(54) **Software distribution and maintenance system for implantable medical devices**

(71) Applicant: BIOTRONIK CRM Patent AG, 6340 Baar (CH)
(72) Inventor: Potschadtke, Jens, 91056 Erlangen (DE); Fournie, Eric, 91054 Erlangen (DE); Merlin, Julian, 10713 Berlin (DE); Bode, Sven, 12203 Berlin (DE)
(74) Representative: Eisenführ, Speiser & Partner

(57) **Abstract**

The invention is directed to a server-client system comprising a central data server and at least one network element at least temporarily connected to the central server via a data communication network to enable a data transmission/communication between the central server and the network element. The network element comprises at least one software element determining the functionality of the network element at least partly. The central server comprises a software storage means containing actual versions of the software elements installed on the network element, a network element configuration database and a software update engine. The configuration database contains a plurality of data elements individually assigned to each network element. The data elements comprise a network element identification element (NWE-ID) for each network element and associated to said network element identification element a list of software element identification elements (SWE-ID), each such software element identification element identifying a software element installed on the network element which is unambiguously identified by said network element identification element and associated to each software element identification element a version identification element (V-ID) identifying the version number of the software element which is identified by the respective software element identification element. The software update engine has access to the configuration database and at least temporarily to the network element. The software update engine is adapted to track the version identification elements associated to the network element, to determine whether a software element installed on the network element has an outdated version number and to initiate a software update on a network element upon determination that a software element installed on the network element has an outdated version number.

## Description

The invention relates to a server-client system comprising a central data server and at least one network element which is at least temporarily connected to central server via a data communication network. The data communication network may be any public or private network like a wide area network or a public telecommunication network, e.g. a telephone network or a wireless cellular network.

The network element comprises at least one software element determining the functionality of the network element at least partly.

One particular embodiment of such server-client system is given by a so called patient device or bedside device which may be permanently or temporarily linked to a central server in a medical care center. The patient device in such embodiment comprises a wireless interface for enabling a data communication between an implanted medical device of a patient and the patient device. This wireless communication link serves for transmitting medical or operational data from the implantable medical device to the patient device. On the other hand the patient device is linked or can be linked to a remote central server. The remote central server is adapted to evaluate data transmitted by the patient device to the remote central server in order to determine a particular health state of a patient or an operational state of the implanted medical device.

In such environment, the telecommunication link may be initiated by either the patient device or the remote central server. The period of time for which an active data telecommunication link exists between the network element (e. g. patient device) and the central server shall be called "access session" further herein. Thus, an access session may be initiated by the network element or the central server.

In the environment described here before not only the patient device but also the implanted medical device (or implantable medical device, IMD) can be considered a network element since both comprise software components or software elements which determine the functionality of the respective device.

In particular in such medical environment as described above updating a software element of a patient device or even more of an implantable medical device is a crucial task which is not left to be done by the patient. Therefore, a software update usually is carried out by the manufacturer in the manufacturers own premises. A decentral software update may be done by sending a technician to maintain the device to be updated.

All in all software updating of a network element in such medical environment is a critical task requiring a lot of effort.

Therefore it is an object of the invention to provide for a solution for facilitating a software update with less effort.

According to the invention the object is achieved by a central server comprising
- a software storage means containing actual versions of the software elements installed on the network element,
- a network element configuration data base, the configuration data base contains a plurality of data elements individually assigned to each network element, the data elements of the configuration data base comprise:
   - a network element identification element for each network element and
   - associated to said network element identification element a list of software element identification elements, each such software element identification element identifying a software element installed on the network element which is unambiguously identified by such said network element identification element and
   - associated to each software element identification element a version identification element identifying the version number of the software element which is identified by the respective software element identification element
- and a software update engine which has access to the configuration data base and at least temporarily to the network element,
   - the software update engine is adapted to track the version identification elements associated to the network element to determine whether a software element installed on the network element has an outdated version number and to initiate a software update on a network element upon determination that a software installed on the network element has an outdated version number.

It is to be noted, that initiation of a software update on a network element by the software update engine does not necessarily imply an immediate initiation of an access session. In fact, such software update may be delayed until a next access session to the respective network element is established either by the network element itself or by the central server comprising the software update engine or even a third network element.

The invention is based on the general idea to create a server-client system which is adapted to carry out an automatic software update on a network element. For this purpose, a central server is equipped with a network element configuration data base and a software update engine which together provide for the functionality to enable an automatic software update on a network element.

In a preferred embodiment, the configuration data base further comprises a software element update indicator assigned to one, a plurality or all network elements, the software element update indicator causing the software update engine to initiate a software update on that network element the software element update indicator is assigned to when this network element eventually is accessed. The software element update indicator allows for a delayed software update if the software update engine determines that a software update should be carried out on a network element. Thus, in a preferred embodiment the software element update indicator is generated automatically by the software update engine. Generation of software element update indicator is performed by the software update engine by way of a comparison of software element version numbers, one of the version numbers indicating the software element version installed on the network element and the other version number indicates an actual software element version. Thus, a software element update indicator can be set by the software update engine prior to accessing a particular network element. The actual software update may be carried out, when the network element, the software element update indicator is assigned to eventually is accessed.

In particular for the latter mentioned comparison in a preferred embodiment the configuration data base further comprises a latest version identification element for some or all software elements which are represented by respective software element identification elements in the configuration data base.

Since it may be beneficial not to always update all software elements on all network elements to the latest version of a particular software element, the configuration data base may further comprise a wanted-configuration sub data base assigned to one or more of the network elements. The wanted-configuration sub data base contains software element identification elements and version identification elements assigned to said software element identification elements characterizing a wanted configuration of the respective network element.

With respect to the latter preferred embodiment, it is further preferred to provide for a software update engine which is adapted to compare an actual configuration of a network element to a wanted configuration of said network element by comparing the software element identification elements and version identification elements of an actual configuration of the software identification elements and the version identification elements stored in the wanted-configuration sub data base to conduct a software update on a network element if the actual software element identification elements and version identification elements differ from the respective once stored in the wanted - configuration sub data base. Thus, the software update engine is able to generate a software element update indicator in the above described manner in a preferred embodiment.

A further preferred embodiment is directed to the updating of the configuration data base. In a preferred embodiment, such update of the configuration data base is carried out by the software update engine each time a data link is established between a respective network element and the central server. Thus, the configuration data base should always represent the latest software element configuration of a particular network element.

A further preferred embodiment is directed to the process of updating one or more software elements on a particular network element. Such update may be carried out during one access session. Alternatively, the software update engine may be adapted to conduct a software update on a network element by transmitting a software element to be updated to the network element in several parts during a plurality of access sessions. A further preferred variant of such embodiment comprises a network element configuration data base further comprising an update status indicator element indicating which parts of a software element have already be transmitted to a network element and whether the transmission of a software element has already been completed or not. Furthermore, a transmission check element in the software element configuration data base may be provided which indicates successful and correct transmission of a complete software element to a network element.

With respect to the latter mentioned preferred embodiment of the software update engine being able to conduct a software update in a plurality of parts, the software update engine preferably is adapted to adjust the size of each part according to a data rate available during an individual access session or based on the reliability of the link established during said access session.

Verification of a correct transmission and/or storage of a part or a complete software element on a network element is preferably carried out by the software update engine. Therefore, a preferred embodiment comprises a software update engine which is adapted to verify a correct transmission and/or storage of a part or a complete software element on a respective network element after transmission of said complete or partial software element. Thus, the software update engine is able to generate the above mentioned update status indicator element and/or the above mentioned transmission check element.

In a preferred embodiment, transmission of a software element to a network element and activation of said software element on the network element may be separated from on another. Thus, a software element may be transmitted to a network element without being activated immediately after transmission. Thereby, a software element can first be checked for completeness prior to activation. Even more, a software element providing for further functionality can already be downloaded to a network element and can be activated later only if the extra functionality provided by the software element actually is needed. Thus, software element conflicts can be avoided to as much an extent as possible without unnecessarily limiting the functionality of a network element. In such case, the network element configuration data base may also comprise a software element activation indicator for each software element stored on a network element. Activation of a software element may be initiated by the software update engine by sending a software activation signal to the respective network element.

Since it may turn out that a software element is not needed anymore on a network element a preferred embodiment of the software update engine is adapted to remove a software element from a network element. In that case, the network element configuration data base further comprises a software element removal indicator assigned to both, a network element and a software element previously installed on that network element, the software element removal indicator causing the software update engine to initiate a software element removal from the respective network element when said network element is accessed the next time.

Instead of removing a complete software element by for example overwriting the according memory on the network element the software update engine may be adapted to disable a software element of a network element without removing the software element by transmitting a disable signal to the network element. When such disable signal is sent to the network element, the software update engine may be update the software element activation indicator to indicate that the respective software element is deactivated.

Deactivating a software element on a network element without removing the software element on said network element allows for later reactivation of said software element. Therefore a software update engine is preferred which is adapted to restart a disabled software element on a network element by transmitting a restart signal to the network element. Then, the software element activation indicator preferably is updated again to indicate that the software element is activated. The restart signal may be identical to above mentioned activation signal.

In a preferred embodiment of the server-client system the network element comprises a bootstrap loader which is adapted to activate a predetermined number or all software element stored and/or installed on the network element when the bootstrap loader is activated. Thus, activation of one or more software elements is not or only indirectly initiated by the software update engine on the central server since activation is initiated by the bootstrap loader on the network element. In the latter case, the bootstrap loader is adapted to generate an activation signal for all software elements or individual ones. The activation of the bootstrap loader may be triggered by the software update engine, that is remotely, or during power up of the network element or if a scheduled point of time is reached or if a reset of network element is carried out. Activation of the bootstrap loader may be triggered if one or more of the latter conditions arise.

The bootstrap loader itself may be a software element. Therefore, in a preferred embodiment the software update engine is adapted to activate, update or modify the bootstrap loader on a particular network element.

In a preferred embodiment, the network element is a patient device. Said patient device has a wireless interface to a medical device, in particular an implantable medical device such as a cardiac pacemaker or an implantable cardioverter/defibrillator. For accessing the central server, the patient device may have an interface to remote communication network such as a public telecommunication network or a wide area network. The interface to said network may be a modem. The network may be a landline network or a wireless (cellular) network or the like

The invention shall now be described by way of an example and with respect to the figures.
- Figure 1: shows a server client system according to the invention in a simplified representation
- Figure 2: shows a software update engine of a central server of the server-client system represented in figure 1;
- Figure 3: shows a network element configuration database of the central server of figure 1; and
- Figure 4: shows a network element as an example for a client in the server-client system of figure 1

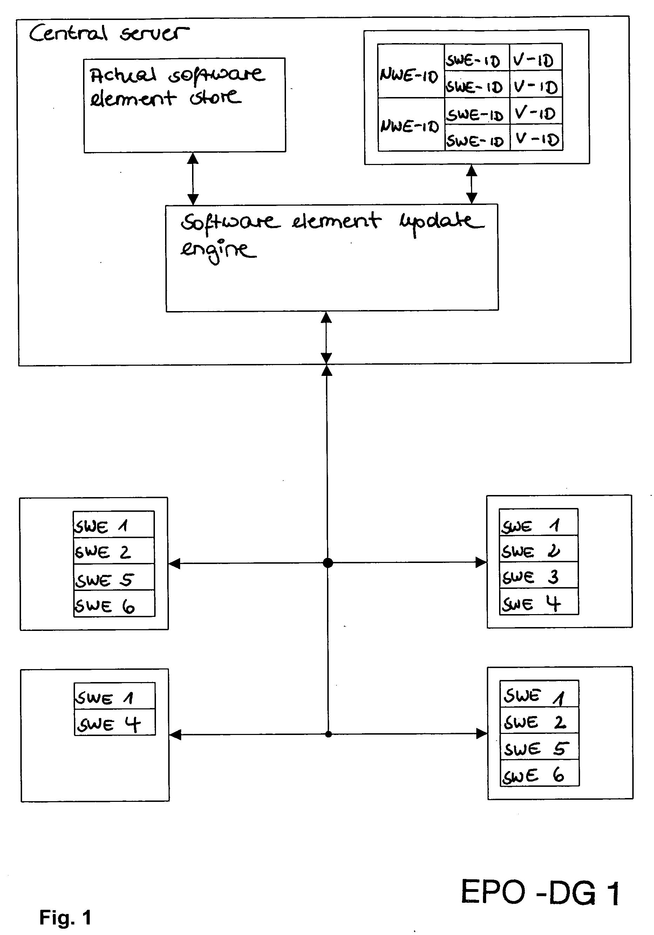

Figure 1 shows a block diagram of a server-client system according to the invention. Main components of said server-client system are a central server 10, a network 12 and a plurality of network elements 14 connected to the central server 10 via the network 12. The connection may be a permanent connection or a temporary connection which is established only during an access session.

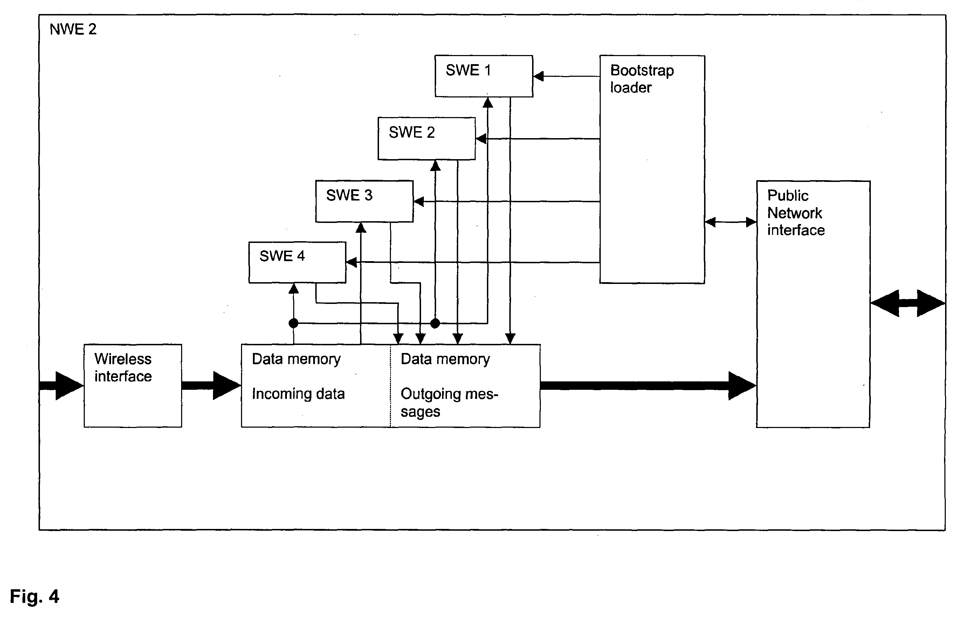

The network elements (see figure 4) comprise software elements (SWE) 16 which are adapted to determine the functionality of the respective network element.

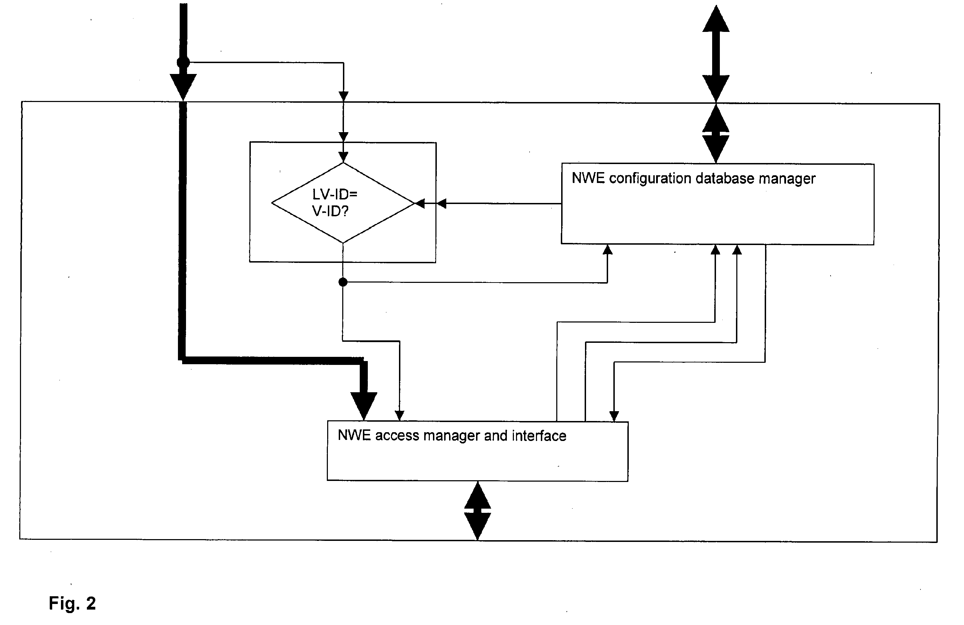

The central server comprises usual components of a computer system including an public network interface (see figure 2) interface to access the network 12.

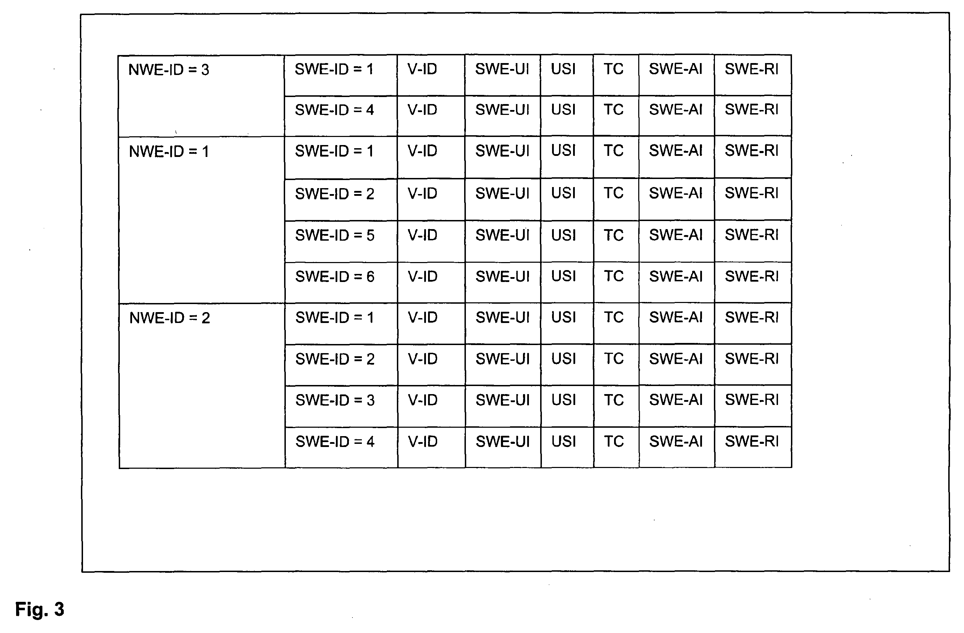

In addition to those general components, the central server 10 comprises a network element configuration data base (NWE-CDB, see figure 3) and a software update engine (SUE; see figure 2). The network element configuration data base includes a wanted-configuration sub data base (WC-SDB, not shown in figure 3).

Basic elements of the network element configuration data base are:
- network element identification element (NEW-ID), each network element identification element identifying an particular network element potentially connected to the central server.
- software element identification elements (SWE-ID) individually assigned to particular network element identification element and identifying the software elements stored or installed on the respective network element.
- a version identification element (V-ID) assigned to each software element identification element identifying the version number of the software element identifying by the according network element identification element and software element identification element, the version identification element is assigned to.

Further elements of the network element configuration data base are:
- a software element update indicator (SWE-UI) indicating to the software update engine, that a software update shall be carried out on the network element the software element update indicator is assigned during the next access session giving he central server access to said network element.
- an update status indicator (USI) element indicating the update status of a software element. The update status indicator element is particularly useful if an update is carried out in a plurality of parts during a number of access sessions.
- a transmission check element (TC) representing a successful transmission and/or installation of a software element or a software element update on a respective network element. The transmission check element may be generate as result of positively checking the checksum of transmitted data representing the software element or the software element update.
- a software element activation indicator element (SWE-AI) assigned to each individual software element identification element and indicating that the respective software element is activated, that is, is actually running determining the functionality of the according network element.
- and a software element removal indicator (SWE-RI) assigned to an individual software element indicating to the software update engine, that a software element shall be removed from the network element the software element update indicator is assigned during the next access session giving he central server access to said network element.

Furthermore, a latest version identification (LV-ID) element may be part of the network element configuration database or of a separate actual software element store. The latest version identification element indicates the version number the latest available version of each software element and thus allows the software update engine to compare a version identification element with a latest version identification element assigned to the same software element identification element in order to determine whether a software element designated by the software element identification element already is up to date or qualifies for an update. In the latter case the software update engine generates a software update indicator for said software element.

It is to be noted, that the latest version identification element is valid for all software element identification elements of the same type whereas the (actual) version identification element is valid for a software element on a particular network element, since same type software elements on different network elements may have different version numbers.

If the software update engine once has generated a software update indicator assigned to a particular software element identification element which in turn is assigned to a particular network element, the software update engine will initiate a software update of that software element on the respective network element during the next access session to said network element.

During such access session a software update is carried out by downloading data representing an actual software element designated by a respective software element identification element to the network element.

For this purpose, a actual software element store is provided an the central server. In that store data representing the latest version of each software element is store in association to a respective software element identification element.

During one or more access sessions data representing an updated software element is transmitted to a respective network element and stored on that network element.

Installation and activation of the updated software element may occur automatically after a transmission is completed and successful transmission is checked. If the data check has led to the result that the transmission was successful, a transmission check element is generated and will be assigned to the respective network identification element and software element identification element.

Alternatively, activation of an updated software element successfully stored on a network element may not immediately be activated but left for later activation via an activation signal. In the preferred embodiment, the software update engine generates the activation signal when activation of an updated software element eventually becomes due.

Furthermore, activation of a software element may be initiated by the network element itself. For this purpose, the network element may comprise a bootstrap loader that is adapted to activate some or all software elements stored on the respective network element. The bootstrap loader itself may be activated or started upon each power up or reset of the network element. Alternatively or additionally the bootstrap loader may be activated by a respective activation signal generated and transmitted by the software update engine.

In a similar manner, the software update engine may a disable signal for an individual software element on an individual network element to switch of or deactivate said software element. The deactivated software element may later be reactivated by a restart signal put out by the software update engine. The restart signal may in fact be identical to the activation signal.

Whether an individual software element on a particular network element is activated or deactivated is indicated in the network element configuration database by a software activation indicator element (SWE-AI) of the configuration database.

An example for a network element according to the invention is depicted in figure 2. The network element (NWE 2) served as client in the server-client system shown in figure 1. The network element is a patient device which serves for receiving data from an implantable medical device via a wireless interface and for sending messages to a central service center including the central server, said messages including information with respect to the health state of the patient or the operational state of the implantable medical device.

Medical or operational data from the implantable medical device is received by the network element via the wireless interface. The data is written into a data memory and evaluated by software elements (SWE 1 to SWE 4). The software elements are adapted to read incoming data from the data memory and to create messages to be sent to the central server via a public network. Thus, the software elements are adapted to write data representing outgoing messages into the data memory. During an access session a public network interface of the network element accesses the data memory to read out the outgoing messages or to transmit these messages to the central server.

With respect to the invention, it is to be noted, that the network element comprises a bootstrap loader which itself may be a software element. The bootstrap loader is adapted to disable or to enable one or more of the software elements. For this purpose, the bootstrap loader will receive the according signals from the central server via the public network. These signals include the software element activation signal or a software element removal signal.

The bootstrap loader also is adapted to check whether a software element to be updated is fully transmitted to the network element and to create a transmission check signal, if the software element has successfully transmitted to the network element. Thus, the bootstrap loader is a kind of a master software element controlling the operational software elements SWE 1 to SWE 4, which are adapted to process incoming data or to create message data.

## Claims

1. Central-server for a server-client system comprising a central data server and at least one network element, which is at least temporarily connected to the central server via a data communication network to enable a data transmission/communication between the central server and the network element and which comprises at least one software element determining the functionality of the network element at least partly, and
wherein the central server comprises
a software storage means containing actual versions of the software elements installed on the network element,
a network element configuration database,
the configuration database contains a plurality of data elements individually assigned to each network element, the data elements comprise
- a network element identification element (NWE-ID) for unambiguously identifying a network element and
- associated to said network element identification element a list of software element identification elements (SWE-ID), each such software element identification element identifying a software element installed on a network element which is unambiguously identified by said network element identification element and
- associated to each software element identification element a version identification element (V-ID) identifying the version number of a software element which is identified by the respective software element identification element
and a software update engine, which has access to the configuration database and which is adapted to at least temporarily access a network element,
the software update engine is adapted to track the version identification elements associated to a network element, to determine whether a software element installed on a network element has an outdated version number and to initiate a software update on a network element upon determination that a software element installed on the network element has an outdated version number.

2. Central server according to claim 1 wherein the configuration database further comprises a software element update indicator assigned to one, a plurality or all network elements, the software element update indicator causing the software update engine to initiate a software update on that network element the software element update indicator is assigned to, when this network element eventually is accessed.

3. Central server according to claim 1 wherein the configuration database further comprises a latest-version identification element (LV-ID) for some or all software elements represented by according software element identification elements (SWE-ID) in the configuration database.

4. Central server according to claim 1 wherein the configuration database further comprises a wanted-configuration sub database assigned to a single network element or a plurality of network elements or all network elements containing software element identification elements (SWE-IDs) and version identification elements (V-IDs) assigned to the software element identification elements (SWE-IDs) characterizing a wanted configuration of a network element.

5. Central server according to claim 4 wherein the software update engine is adapted to compare an actual configuration of a network element to a wanted configuration of said network element by comparing the software element identification elements (SWE-IDs) and version identification elements (V-IDs) of an actual configuration to the software element identification elements (SWE-IDs) and version identification elements (V-IDs) stored in the wanted-configuration sub database and to conduct a software update on a network element if the actual software element identification elements (SWE-IDs) and version identification elements (V-IDs) differ from the according ones stored in the wanted-configuration sub database.

6. Central server according to claim 1 wherein the software update engine is adapted to update the configuration database of the central server each time the central server accesses a network element.

7. Central server according to claim 1 wherein the software update engine is adapted to conduct a software update on a network element by transmitting a software element to be updated in several parts to the network element during a plurality of access sessions.

8. Central server according to claim 7 wherein the software update engine is adapted to adjust the size of each part according to a data rate available during an individual access session.

9. Central server according to one of claims 1 to 8 wherein the software update engine is adapted to verify correct transmission and/or storage of a part or a complete software element on a network element after transmission of said complete or partial software element.

10. Central server according to claim 1 wherein the software update engine is adapted to activate a software element stored and/or installed on a network element independently from a transmission of a software element update to said network element by transmitting a software element activation signal in a separate access session.

11. Central server according to claim 1 wherein the software update engine is adapted to remove a software element from a network element during an access session and wherein the configuration database further comprises a software element removal indicator assigned to both, a network element and software element previously installed on that network element, the software element removal indicator causing the software update engine to initiate a software element removal from the network element, when this network element is accessed the next time.

12. Central server according to claim 1 wherein the software update engine is adapted to disable a software element of a network element without removing the software element by transmitting a disable signal to the network element.

13. Central server according to claim 12 wherein the software update engine is adapted to restart a disabled software element on a network element by transmitting a restart signal to the network element.

14. Network element for a server-client system comprising a central data server and at least one network element, which is adapted to be at least temporarily connected to the central server via a data communication network to enable a data transmission/communication between the central server and the network element and which comprises at least one software element determining the functionality of the network element at least partly, and the network element comprises a bootstrap loader which is adapted to activate a predetermined number or all software elements stored and/or installed on the network element when the bootstrap loader is activated.

15. Network element according to claim 14 wherein a network element is adapted to activate the bootstrap loader under predetermined conditions, the conditions being at least one of: power-up of the network element, scheduled point of time, receiving of a bootstrap loader activation signal, and/or a reset of the network element.

16. Network element according to claim 14 or 15 wherein the software update engine is adapted to update or modify the bootstrap loader.

17. Network element according to one of claims 14 to 16 wherein the network element is a patient device having an interface to a communication network or wireless network connected to the central server and a wireless interface to a medical device, in particular an implantable medical device such as a cardiac pacemaker or an implantable cardioverter/defibrillator.

18. Network element according to one of claims 14 to 16 wherein the network element is a patient device having a modem interface to a telecommunication network for accessing the central server and a wireless interface to a medical device, in particular an implantable medical device such as a cardiac pacemaker or an implantable cardioverter/defibrillator.

19. Server-client system according to one of claims 1 to 18, comprising a network element according to one of claims 14 to 18 and a central server according to one of claims 1 to 13.
